(19) Europäisches Patentamt
European Patent Office
Office européen des brevets

(11) EP 1 285 069 B1

(12) FASCICULE DE BREVET EUROPEEN

(45) Date de publication et mention de la délivrance du brevet:
**13.08.2008 Bulletin 2008/33**

(21) Numéro de dépôt: **01938357.9**

(22) Date de dépôt: **28.05.2001**

(51) Int Cl.:
***C12N 15/31*** (2006.01) ***C07K 14/335*** (2006.01)
***A23L 3/3463*** (2006.01) ***A23L 3/3571*** (2006.01)

(86) Numéro de dépôt international:
**PCT/FR2001/001642**

(87) Numéro de publication internationale:
**WO 2001/092533 (06.12.2001 Gazette 2001/49)**

# (54) BACTERIOCINE ANTI-LISTERIA

BAKTERIOCIN GEGEN LISTERIA

ANTI-LISTERIA BACTERIOCIN

(84) Etats contractants désignés:
**AT BE CH CY DE DK ES FI FR GB GR IE IT LI LU MC NL PT SE TR**

(30) Priorité: **29.05.2000 FR 0006859**
**19.10.2000 FR 0013407**

(43) Date de publication de la demande:
**26.02.2003 Bulletin 2003/09**

(60) Demande divisionnaire:
**08101852.5 / 1 927 661**

(73) Titulaire: **DANISCO A/S**
**1411 Copenhagen K. (DK)**

(72) Inventeurs:
- **BERJEAUD, Jean-Marc**
**F-86800 Savigny l'Evescault (FR)**
- **FREMAUX, Christophe**
**F-86600 Poitiers (FR)**
- **CENATIEMPO, Yves**
**F-86800 Saint Julien l'Ars (FR)**
- **SIMON, Laurence**
**F-86370 Vivonne (FR)**

(74) Mandataire: **Nargolwalla, Cyra et al**
**Cabinet Plasseraud**
**52 rue de la Victoire**
**75440 Paris Cedex 09 (FR)**

(56) Documents cités:
- **HUGAS M ET AL: "Application of the bacteriocinogenic Lactobacillus sakei CTC494 to prevent growth of Listeria in fresh and cooked meat products packed with different atmospheres" FOOD MICROBIOL., vol. 15, 1998, pages 639-650, XP000982835 ISSN: 0021-8847**
- **AXELSSON L ET AL: "THE GENES INVOLVED IN PRODUCTION OF AND IMMUNITY TO SAKACIN A, A BACTERIOCIN FROM LACTOBACILLUS SAKE LB706" JOURNAL OF BACTERIOLOGY, US,WASHINGTON, DC, vol. 177, no. 8, 1 avril 1995 (1995-04-01), pages 2125-2137, XP000673873 ISSN: 0021-9193**
- **HUEHNE KATHRIN ET AL: "Analysis of the sakacin P gene cluster from Lactobacillus sake Lb674 and its expression in sakacin-negative Lb. sake strains." MICROBIOLOGY (READING), vol. 142, no. 6, 1996, pages 1437-1448, XP000982832 ISSN: 1350-0872**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne une bactériocine de *Lactobacillus sakei* et plus particulièrement de *Lactobacillus sakei* 2512, une séquence nucléotidique codant pour cette bactériocine, et l'utilisation industrielle de cette bactériocine comme agent actif contre des flores pathogènes ou indésirables dans la préparation de produits alimentaires.

Les bactéries lactiques sont utilisées intensivement dans les fermentations alimentaires afin, non seulement d'améliorer la saveur et la texture des aliments mais surtout pour allonger leur durée de conservation. De nombreuses bactéries lactiques ont en effet la faculté d'inhiber la croissance de certaines bactéries à Gram positif, dont des souches pathogènes comme *Listeria monocytogenes,* grâce à l'excrétion de molécules antagonistes, parmi lesquelles des composés peptidiques. Ces composés peptidiques, appelés bactériocines, présentent donc un potentiel intéressant pour la préservation qualitative et sanitaire de produits alimentaires fermentés.

A titre représentatif de ces bactériocines, on peut notamment citer celles formant la sous-classe de polypeptides dénommés bactériocines *anti-Listeria,* bactériocines de classe IIa (Ennahar S. et al., 2000, FEMS Microbiol. Rev., 24 : 85-106) et cystibiotiques (Jack R. et al., 1995, Microbiol. Rev., 59(2) :171-200). Il a été fait récemment état de l'utilisation potentielle d'une de ces bactériocines de classe IIa, la divercine V41, pour empêcher la croissance de *Listeria monocytogenes* dans du saumon fumé (Duffes F. et al., 1999, J. Food Prot., 62(12) :1394-1403).

Les séquences de ces polypeptides présentent de fortes similitudes dans leur partie N-terminale, avec en particulier la présence d'un pont disulfure. La partie C-terminale hydrophobe est beaucoup plus variable, toutefois certaines de ces bactériocines, dites de type pédiocine (pédiocine PA-1, entérocine A et divercine V41), se caractérisent par une taille supérieure à 40 résidus et la présence d'un deuxième pont disulfure du coté C-terminal.

Les auteurs de la présente invention ont mis en évidence une nouvelle bactériocine de classe IIa produite à partir d'une souche spécifique de *Lactobacillus sakei,* qui s'avère particulièrement efficace pour inhiber la croissance de *Listeria,* plus particulièrement de *Listeria monocytogenes.*

En accord avec Tagg J.R, et al., Bacteriol. Rev., 40 ; 722-756 (1976), le terme " Bactériocine " au sens de l'invention fait référence à un polypeptide produit, par synthèse ribosomique, à partir de microorganismes, capable d'inhiber spécifiquement la croissance d'autres bactéries,

Hugas et al., Food Microbiol, vol. 15, 1998, pages 639-650 décrit l'isolement de la Sakacine K de Lactobacillus Sakei CTC 494 et son utilisation pour empêcher la croissance et la propagation de Listeria monocytogènes dans des produits alimentaires.

La présente invention a donc pour premier objet un polypeptide issu de la souche *Lactobacillus sakei* 2512, doté d'une activité bactériocine.

La souche *Lactobacillus sakei* 2512 a été déposée le 25 mai 2000 auprès de la Collection Nationale des Cultures de Microorgsnismes où elle est enregistrée sous le numéro de dépôt I-2479.

La bactériocine objet de la présente invention a été dénommée Sakacine G. Il s'agit d'un polypeptide possédant une masse moléculaire de l'ordre de 3700 à 3900 et préférentiellement d'environ 3834 Da déterminée par spectrométrie de masse. Elle possède un spectre d'inhibition bactérienne très apparenté à celui des bactériocines de Classe IIa. C'est ainsi qu'elle s'avère particulièrement efficace contre les souches de *Lactobacillus sakei* autres que le *Lactobacillus sakei* 2512, *Pediococcus cerevisiae,* l'ensemble des souches *Listeria* et contre les *Enterococcus faecalis* et *durans.* En revanche, elle s'avère inactive contre les autres espèces de *Lactobacillus* comme par exemple le *Lactobacillus debrueckii,* le *Lactobacillus plantarum,* le *Lactobacillus brevis,* le *Lactobacillus casei,* et une souche *d'Enterococcus faecium.*

A l'image des bactériocines anti-*Listeria* de type pédiocine, la Sakacine G possède dans sa structure peptidique avantageusement deux ponts disulfures.

Une analyse des déterminants génétiques de plusieurs bactériocines de classe IIa a montré que les gènes impliqués dans leurs production, transport et immunité, sont organisés en une ou plusieurs structures de type opéron. Ces opérons ont une localisation souvent plasmidique et possèdent généralement au moins deux gènes codant pour des protéines, homologues à un ABC-transporteur et une protéine accessoire, probablement impliquée dans l'export des bactériocines.

Le clonage du fragment nucléotidique contenant le gène de la Sakacine G a révélé l'existence de trois cadres ouverts de lecture complets *skgA1* (SEQ ID N°1), *skgA2* (SEQ ID N°3) et *skgDc* (SEQ ID N°13) (incluant le cadre de lecture tronqué *skgD* (SEQ ID N°7)) et d'un cadre tronqué *skgI* (SEQ ID N°5) dont une représentation schématique est présentée en figure 1. Le fragment nucléotidique est un double brin dont le monobrin 5'-3' est représenté en séquence ID N°15.

Les produits des gènes *skgA1* et *skgA2,* appelés pré-bactériocines, peuvent subir une maturation au cours de laquelle leurs peptides leaders respectifs sont clivés entre les résidus 18 et 19, libérant ainsi la Sakacine G active (résidus 19-55).

Le fragment nucléotidique monobrin 5'-3' comprenant *skgA1, skgA2, skgD* et *skgI* figure en SEQ ID N°9.

La présente invention a donc également pour objet un polypeptide isolé correspondant à une bactériocine, caractérisé en ce qu'il comprend la séquence ID N°2 et/ou la séquence ID N°4. La séquence de la bactériocine mature correspond à la séquence ID N°12 et est comprise dans les séquences ID N°2 et ID N°4.

Le cadre de lecture appelé *skgI* code une protéine de 52 résidus. La comparaison de cette séquence avec celle des banques de données montre de fortes similitudes de SkgI avec des protéines dites d'immunité. Elle code vraisemblablement la protéine d'immunité protégeant la bactérie productrice de la Sakacine G.

La présente invention s'étend également à un polypeptide isolé comprenant la séquence ID N°6 correspondant au cadre de lecture *skgI.*

En ce qui concerne le dernier gène *skgDc,* il code une protéine qui présente une homologie avec des protéines de la famille des ABC-transporteurs, et plus particulièrement du transporteur de la pédiocine PA-1. Le gène *skgDc* code vraisemblablement l'ABC-transporteur spécifique de la Sakacine G.

La présente invention s'étend également au polypeptide isolé comprenant la séquence ID N°8 correspondant au gène dit *skgD* et au polypeptide isolé comprenant la séquence ID N°14 correspondant au gène dit *skgDc.*

Il est entendu que sont également comprises les séquences homologues, définies comme

i) les séquences similaires à au moins 70% de la séquence SEQ ID N° 2, N° 4, N°6, N°8, N°12, ou N°14 ; ou
ii) les séquences codées par une séquence d'acide nucléique homologue telle que définie ci-après c'est-à-dire une séquence d'acide nucléique hybridant avec la séquence SEQ ID N° 1, N° 3, N° 5, N° 7, N° 9, N°13 ou N°15 ou sa séquence complémentaire, dans des conditions stringentes d'hybridation.

Là encore, le terme "similaires" se réfère à la ressemblance parfaite ou identité entre les acides aminés des séquences homologues comparées mais aussi à la ressemblance non parfaite que l'on qualifie de similitude. Cette recherche de similitudes dans une séquence polypeptidique prend en compte les substitutions conservatives qui sont des substitutions d'acides aminés de même classe, telles que des substitutions d'acides aminés aux chaînes latérales non chargées (tels que l'asparagine, la glutamine, la serine, la thréonine, et la tyrosine), d'acides aminés aux chaînes latérales basiques (tels que la lysine, l'arginine, et l'histidine), d'acides aminés aux chaînes latérales acides (tels que l'acide aspartique et l'acide glutamique); d'acides aminés aux chaînes latérales apolaires (tels que la glycine, l'alanine, la valine, la leucine, l'isoleucine, la proline, la phénylalanine, la méthionine, le tryptophane, et la cystéine).

Plus généralement, par " séquence d'acides aminés homologue ", on entend donc toute séquence d'acides aminés qui diffère de la séquence SEQ ID N°2, N°4, N°6, N°8, N°12 ou N°14 par substitution, délétion et/ou insertion d'un acide aminé ou d'un nombre réduit d'acides aminés, notamment par substitution d'acides aminés naturels par des acides aminés non naturels ou pseudo-acides aminés à des positions telles que ces modifications ne portent pas significativement atteinte à l'activité biologique du polypeptide isolé et de préférence de la Sakacine G.

De préférence, une telle séquence d'acides aminés homologue est similaire à au moins 85 % de la séquence SEQ ID N°2, N°4, N°6, N°8, N°12 ou N°14, de préférence au moins 95 %.

L'homologie est généralement déterminée en utilisant un logiciel d'analyse de séquence (par exemple, Sequence Analysis Software Package of the Genetics Computer Group, University of Wisconsin Biotechnology Center, 1710 University Avenue, Madison, WI 53705). Des séquences d'acides aminés similaires sont alignées pour obtenir le maximum de degré d'homologie (i.e. identité ou similitude, comme défini plus haut). A cette fin, il peut être nécessaire d'introduire de manière artificielle des espaces (" gaps ") dans la séquence. Une fois l'alignement optimal réalisé, le degré d'homologie est établi par enregistrement de toutes les positions pour lesquelles les acides aminés des deux séquences comparées sont identiques, par rapport au nombre total de positions.

L'activité biologique du polypeptide isolé et notamment de la Sakacine G se réfère à sa capacité à inhiber la croissance de souches bactériennes indésirables et/ou pathogènes, de préférence de bactéries *Listeria* et plus particulièrement de bactéries *Listeria monocytogenes.*

La présente invention a également pour objet un acide nucléique isolé, codant pour un polypeptide tel que défini précédemment.

Plus précisément, la présente invention a pour objet un acide nucléique isolé comprenant la séquence ID N°1 et/ou la séquence ID N°3.

La séquence nucléotidique complète de la région impliquée dans l'expression de la Sakacine G (3055 pb) a été déterminée. Il s'agit d'un ADN double brin dont le brin 5'-3' est représenté en séquence ID N°15. Le brin 3'-5' est présenté en figure 2. La présente invention vise également un acide nucléique comprenant une telle séquence.

Comme décrit précédemment, cette séquence possède trois cadres ouverts de lecture complets *skgA1, skgA2* et *skgDc* et un tronqué, *skgI.* Les gènes supposés *skgA1* (SEQ ID N°1), *skgA2* (SEQ ID N°3) et *skgI* (SEQ ID N°5) y sont orientés en sens inverse par rapport à *skgDc* (SEQ ID N°13).

Sont également revendiqués dans le cadre de la présente invention, l'acide nucléique de séquence ID N°5, l'acide nucléique de séquence ID N°13 et l'acide nucléique de séquence ID N°7.

Il est entendu que sont également comprises les séquences homologues, définies comme :

i) des séquences similaires à au moins 70 % de la séquence SEQ ID N°1, N°3, N°5, N°7, N°9, N°13 ou N°15 ; ou
ii) des séquences hybridant avec la séquence SEQ ID N°1, N°3, N°5, N°7, N°9, N°13 ou N°15 ou leur séquence complémentaire, dans des conditions stringentes d'hybridation, ou
iii) des séquences codant pour le polypeptide dénommé Sakacine G, tel que défini précédemment.

De préférence, une séquence nucléotidique homologue selon l'invention est similaire à au moins 75 % des séquences SEQ ID N°1, N°3, N°5, N°7, N°9, N°13 ou N°15, de préférence encore au moins 85 %, ou au moins 90 %.
De manière préférentielle, une telle séquence nucléotidique homologue hybride spécifiquement aux séquences complémentaires de la séquence SEQ ID N°1, N°3, N°5, N°7, N°9, N°13 ou N°15 dans des conditions stringentes. Les paramètres définissant les conditions de stringence dépendent de la température à laquelle 50% des brins appariés se séparent (Tm).
Pour les séquences comprenant plus de 30 bases, Tm est définie par la relation (Sambrook *et al.,* 1989,NY.: Cold Spring Harbor Laboratory) :

$$Tm = 81{,}5 + 0{,}41(\%G{+}C) + 16{,}6\ Log(\text{concentration en cations}) - 0{,}63(\%\text{formamide}) - (600/\text{nombre de bases})$$

Pour les séquences de longueur inférieure à 30 bases, Tm est définie par la relation :

$$Tm = 4(G{+}C) + 2\ (A{+}T).$$

Dans des conditions de stringence appropriées, auxquelles les séquences aspécifiques n'hybrident pas, la température d'hybridation peut être de préférence de 5 à 10°C en dessous de Tm, et les tampons d'hybridation utilisés sont de préférence des solutions de force ionique élevée telle qu'une solution 6xSSC par exemple.
Le terme "séquences similaires" employé plus haut se réfère à la ressemblance parfaite ou identité entre les nucléotides comparés mais aussi à la ressemblance non parfaite que l'on qualifie de similitude. Cette recherche de similitudes dans les séquences nucléiques distingue par exemple les purines et les pyrimidines.
Une séquence nucléotidique homologue aux cadres ouverts de lecture représentés en SEQ ID N°1, N°3, N°5, N°7, N°9, N°13 ou N°15 inclut donc toute séquence nucléotidique qui diffère de la séquence SEQ ID N°1, N°3, N°5, N°7, N°9, N°13 ou N°15 par mutation, insertion, délétion ou substitution d'une ou plusieurs bases, ou par la dégénérescence du code génétique, pour autant qu'elle code un polypeptide présentant l'activité biologique de la Sakacine G, comme définie ci-après.
Parmi de telles séquences homologues, sont comprises les séquences des gènes de bactéries autres que *Lactobacillus,* codant pour la Sakacine G.
Les polypeptides de la présente invention peuvent être synthétisés par toutes les méthodes bien connues de l'homme du métier. Les polypeptides de l'invention peuvent par exemple être synthétisés par les techniques de la chimie de synthèse, telles que la synthèse de type Merrifield qui est avantageuse pour des raisons de pureté, de spécificité antigénique, d'absence de produits secondaires non désirés et pour sa facilité de production.
La présente invention a également pour objet un procédé de production d'un polypeptide recombinant dans lequel un vecteur comprenant un acide nucléique conforme à la présente invention est transféré dans une cellule hôte qui est mise en culture dans des conditions permettant l'expression d'un polypeptide conforme à la présente invention ou d'un polypeptide codé par une séquence d'acide nucléique conforme à la présente invention.
La bactériocine recombinante peut également être produite par un procédé, dans lequel un vecteur contenant un acide nucléique comprenant une séquence nucléotidique conforme à l'invention et de préférence les séquences SEQ ID N°1 et/ou N°3 où une séquence homologue est transférée dans une cellule hôte qui est mise en culture dans des conditions permettant l'expression du polypeptide correspondant. La protéine produite peut ensuite être récupérée et purifiée. Les procédés de purification utilisés sont connus de l'homme du métier. Le polypeptide recombinant obtenu peut être purifié à partir de lysats et extraits cellulaires, du surnageant du milieu de culture, par des méthodes utilisées individuellement ou en combinaison, telles que le fractionnement, les méthodes de chromatographie, les techniques d'immunoaffinité à l'aide d'anticorps mono- ou polyclonaux spécifiques, etc.
La séquence d'acide nucléique d'intérêt, codant pour la Sakacine G, peut être insérée dans un vecteur d'expression, dans lequel elle est liée de manière opérante à des éléments permettant la régulation de son expression, tels que notamment des promoteurs, activateurs et/ou terminateurs de transcription. Les signaux contrôlant l'expression des séquences nucléotidiques (promoteurs, activateurs, séquences de terminaison...) sont choisis en fonction de l'hôte cellulaire utilisé. A cet effet, les séquences nucléotidiques selon l'invention peuvent être insérées dans des vecteurs à réplication autonome au sein de l'hôte choisi, ou des vecteurs intégratifs de l'hôte choisi. De tels vecteurs seront préparés selon les méthodes couramment utilisées par l'homme du métier, et les clones en résultant peuvent être introduits dans un hôte approprié par des méthodes standard, telles que par exemple l'électroporation ou la précipitation au phosphate de calcium.

Les vecteurs de clonage et/ou d'expression tels que décrits ci-dessus, contenant une séquence nucléotidique définie selon l'invention font également partie de la présente invention.

L'invention vise en outre les cellules hôtes transformées, de manière transitoire ou stable, par ces vecteurs d'expression. Ces cellules peuvent être obtenues par l'introduction dans des cellules hôtes, de préférence procaryotes, d'une séquence nucléotidique insérée dans un vecteur tel que défini ci-dessus, puis la mise en culture desdites cellules dans des conditions permettant la réplication et/ou l'expression de la séquence nucléotidique transférée.

Des exemples de cellules hôtes incluent notamment des bactéries telles que *Lactococcus, Lactobacillus, Leuconostoc, Streptococcus, Pediococcus, Escherichia* et les levures.

Les séquences nucléotidiques de l'invention peuvent être d'origine artificielle ou non. Il peut s'agir de séquences d'ADN ou d'ARN, obtenues par criblage de banques de séquences au moyen de sondes élaborées sur la base des séquences SEQ ID N°1, N°3, N°5, N°7, N°9, N°13 et/ou N°15. De telles banques peuvent être préparées par des techniques classiques de biologie moléculaire, connues de l'homme de l'art.

Les séquences nucléotidiques selon l'invention peuvent également être préparées par synthèse chimique, ou encore par des méthodes mixtes incluant la modification chimique ou enzymatique de séquences obtenues par criblage des banques.

La présente invention se rapporte également à un procédé pour inhiber la croissance de *Listeria,* plus particulièrement de *Listeria monocytogenes* dans un environnement qui peut être alimentaire ou non et qui est susceptible d'être contaminé avec les *Listeria monocytogenes.*

Les *Listeria monocytogenes* sont des microorganismes pathogènes qui sont à l'origine de sévères maladies chez les êtres humains et animaux et qui peuvent notamment être facilement transmissibles par des aliments contaminés, plus spécialement au moyen de viandes, de produits carnés, de produits marins, de lait et de produits dérivés. La présente invention propose donc un procédé pour inhiber la croissance de *Listeria monocytogenes* dans un aliment susceptible de contenir des *Listeria monocytogenes* à titre de contaminant, ledit procédé comprenant l'addition d'un polypeptide conforme à l'invention dans ledit aliment en une quantité suffisante pour inhiber la croissance de *Listeria monocytogenes.*

Les bactériocines conformes à l'invention sont de préférence utilisées dans tout système alimentaire en une quantité comprise entre 1 et 100000 unités arbitraires (AU) de bactériocines par gramme d'aliment.

Une AU de bactériocines est définie comme 5 $\mu$l de la dilution la plus élevée du surnageant de culture conduisant à une zone définie d'inhibition de croissance par rapport à une souche témoin d'une bactérie à Gram positif sur un milieu agar.

Bien que les aliments soient les plus concernés par une contamination par *Listeria monocytogenes,* les produits vétérinaires et médicaux peuvent également être contaminés avec ce type de bactéries, de même que les produits cosmétiques ou produits apparentés.

Les bactériocines conformes à la présente invention, et notamment la Sakacine G, sont donc également utiles pour inhiber la croissance de ce type de pathogènes dans ces produits.

La présente invention a ainsi pour objet l'utilisation d'une bactériocine conforme à la présente invention comme agent actif contre des flores pathogènes ou indésirables notamment dans la préparation de produits alimentaires et plus précisément pour inhiber la croissance et la propagation de *Listeria,* plus particulièrement de *Listeria monocytogenes,* dans les produits alimentaires.

Le polypeptide peut être incorporé tel quel dans le produit alimentaire considéré ou encore y être produit à partir de la souche *Lactobacillus Sakei* 2512.

La présente invention a ainsi également pour objet l'utilisation de la souche *Lactobacillus Sakei* 2512 dans un produit alimentaire pour y générer un polypeptide bactériocine conforme à l'invention.

L'invention concerne encore une composition bactériocine, caractérisée en ce qu'elle comprend au moins un polypeptide conforme à la présente invention, c'est-à-dire issue de la souche *Lactobacillus Sakei* 2512 ou comprenant la séquence SEQ ID N°2, ou N°4, ou N°12, ou N°14 ou la souche *Lactobacillus Sakei* 2512.

L'invention s'étend également à l'utilisation de la souche *Lactobacillus sakei* 2512 destinée à produire un polypeptide tel que défini plus haut, pour inhiber la croissance et la propagation de *Listeria,* plus particulièrement de *Listeria monocytogenes,* dans des produits alimentaires ainsi que les compositions comportant de telle souche.

Les exemples et la figure ci-après sont présentés à titre illustratif de l'objet de la présente invention.

FIGURE :

Figure 1 : Représentation schématique du locus génétique impliqué dans la production de la sakacine G.
Figure 2 : Brin complémentaire 3'-5' correspondant à la séquence nucléotidique complète de la région impliquée dans l'expression de la Sakacine G et dont le brin 5'-3' est présenté en SEQ ID N°15.

MATERIELS ET METHODES

• Souches bactériennes et milieux de culture. *Lactobacillus sakei* 2512 est cultivée à 30°C en milieu MRS (DIFCO Laboratories) stérilisé 12 min à 110°C. Les souches indicatrices sont cultivées en milieu BHI ("brain-heart infusion"; DIFCO Laboratories) à 37°C.
• Test d'activité. Du milieu BHI, gélosé à 10g/1, est ensemencé à 1 % par une préculture de souche indicatrice en phase stationnaire avant d'être coulé en boite de Petri. Cinquante microlitres de solution de sakacine G sont déposés dans des puits creusés dans la gélose refroidie à l'emporte pièce. L'activité bactériocine se traduit par l'apparition de zones d'inhibition autour des puits après incubation une nuit à 37°C.
• Analyse protéique. La sakacine G est analysée en spectrométrie de masse sur un appareil Perkin-Elmer Sciex API 165 équipé d'une source d'ionisation par Ionspray. Après lyophilisation, la fraction HPLC active est reprise avec une solution acétonitrile / eau (1:1) contenant 0,1% d'acide formique puis injectée par infusion à un débit de 5 μl/min. La concentration protéique est déterminée par la méthode à l'acide bicinchoninique au moyen du kit BCA (Sigma) selon les instructions du fabriquant.
Les comparaisons de séquences protéiques sont réalisées grâce au programme BLAST (1), accessible à partir du serveur ExPASy du "Swiss Institute of Bioinformatics".
• Clonage moléculaire et transformation. Les plasmides sont extraits et purifiés à partir de souches d' *Escherichia. coli* et de *Lactobacillus sakei* 2512 selon les méthodes décrites précédemment par Sambrook *et al.,* 1989, NY : Cold Spring Harbor Laboratory et Muriana et Klaenhammer, 1987, Appl. Environ. Microbiol., 53 :553-560 respectivement.

Les enzymes de restriction et de modification de l'ADN sont utilisées selon les indications du fournisseur (Gibco-BRL). Les électrophorèses en gel d'agarose, analytique et préparative, sont conduites en tampon Tris/borate/EDTA (pH 8,3) selon les méthodes décrites par Sambrook *et al.,* 1989, NY : Cold Spring Harbor Laboratory. Les fragments d'ADN digérés sont purifiés à partir des gels d'agarose en utilisant le kit "Prep-a-Gene" (Bio-Rad). Les clonages dans les plasmides pGEM-T (Promega) et pZERO2 (Invitrogen) sont réalisés selon les recommandations des fournisseurs. Le transfert de type Southern est réalisé sur membrane de nylon (Hybond-N+, Amersham) selon Sambrook *et al.,* 1989, NY : Cold Spring Harbor Laboratory. Le transfert est suivi d'une hybridation avec une sonde radioactive obtenue par marquage au $^{32}P$ à l'aide du kit "random primers DNA labelling system" (Gibco-BRL). Les bactéries *E. coli* sont rendues compétentes et transformées selon la méthode de Hanahan, 1983. J. Mol. Biol. 166:557-80.
La Taq polymérase (Gibco-BRL) est utilisée selon les recommandations du fournisseur. L'amplification du fragment d'ADN codant la Sakacine G a été réalisée à l'aide d'un appareil "Geneamp 9700®" (Perkin-Elmer) selon les conditions suivantes : 35 cycles de dénaturation à 94°C pendant 30 s, hybridation à 45°C pendant 30 s et élongation à 72°C pendant 1 min suivis d'un cycle supplémentaire d'élongation à 72°C pendant 5 min.
Le fragment d'ADN portant le locus sakacine G est séquencé à l'aide d'un séquenceur automatique ABI Prism 310® (Perkin-Elmer) en utilisant le kit de séquençage "Big-dye terminator®" (Perkin-Elmer) et les amorces nucléotidiques appropriées.

EXEMPLE 1 :

Isolement et purification de la Sakacine G.

Une culture de 16 h de *Lactobacillus. sakei* 2512 (100 ml) est centrifugée à 6000g pendant 15 min. Le surnageant de culture est ensuite chauffé à 70°C pendant 20 min. Le surnageant refroidi est ensuite dilué avec 1 volume d'eau (le pH de la solution diluée doit être inférieur à 6, par addition d'HCl 1M si nécessaire) avant d'être passé sur une colonne (2.5 x 18 cm) contenant une résine échangeuse de cations (carboxy-methyl cellulose; Cellufine C-200, Amicon) équilibrée avec de l'eau. Après des lavages successifs avec de l'eau (100 ml) puis une solution de NaCl 0,1M (150 ml), la Sakacine G est éluée avec une solution de NaCl 0,5M (200 ml). Le pH de toutes les solutions doit être inférieur à 6. La fraction active est ensuite déposée sur cartouche d'extraction en phase solide (Sep-pak plus C18, Waters) équilibrée dans l'eau. Après lavages successifs avec 5 ml de solutions d'acétate d'ammonium 20 mM contenant 0, 10, 20 et 30% d'acétonitrile, la Sakacine G est éluée avec 10 ml d'acétate d'ammonium 20 mM contenant 80% d'acétonitrile. Après lyophilisation, l'extrait est solubilisé dans 1 ml de solution aqueuse d'acétonitrile à 40% puis injecté sur une colonne HPLC analytique de phase inverse en C8 (Kromasil, 5μm, 100 Å, 4.6 x 250 mm, A.I.T.). L'HPLC a été réalisée sur un appareillage comprenant une pompe Perkin-Elmer series 200 LC connectée à un détecteur Perkin-Elmer 785A. Le chromatogramme en absorption est enregistré à 220 nm. La séparation est réalisée, à un débit de 0,8 ml/min selon le gradient suivant : Solvant A = eau/acide trifluoroacétique 0,1%; solvant B = acétonitrile/eau/ acide trifluoroacétique 0,07%. Après un lavage de 5 min avec 20% de solvant B, l'élution est réalisée par un gradient de 20 de 40% de solvant B en 10 min puis de 40 à 55% de solvant B en 20 min.

La fraction correspondant au pic à 23 min s'étant révélée active contre *Listeria ivanovii* BUG 496 a été analysée en spectrométrie de masse en ionisation "ionspray". La molécule apparaît pure à au moins 95% et possède une masse moléculaire de 3834,32 ± 0,31 Da. La quantité de Sakacine G ainsi purifiée a été estimée à 120 μg à partir de 100 ml de culture. Le rendement de purification a été estimé à 55% d'activité retrouvée. Une partie de la séquence primaire de la Sakacine G a été déterminée par microséquençage et deux oligonucléotides dégénérés ont été établis à partir de cette séquence.

<u>EXEMPLE 2 :</u>

Clonage du locus génétique impliqué dans la production de la sakacine G

Par génétique inverse, deux oligonucléotides dégénérés SakG01 (5' AARTATTATGGNAAYGGNGT 3') (SEQ ID N°10) et SakG02S (5' ACATGATGNCCNCCRTTNGC 3') (SEQ ID N°11) ont été choisis afin d'amplifier le fragment d'ADN correspondant au gène de structure de la sakacine G mature (SEQ ID N°15) par réaction de polymérisation en chaîne (PCR). L'amplifiat ainsi obtenu, d'une taille approximative de 100 pb a été cloné dans le plasmide pGEM-T pour former le plasmide pJMBYC01. Le fragment de restriction *Pvu*II de 560 pb issu de pJMBYC01, incluant le fragment inséré, a servi de sonde d'hybridation, lors d'un transfert de type Southern, pour localiser le gène de structure sur le génome de *Lactobacillus sakei* 2512. A partir d'un extrait plasmidique de *Lb. sakei* 2512 digéré par les enzymes de restriction *Hind*III et *EcoR*I, la sonde a révélé des fragments de tailles respectives d'environ 2,1 et 9 kpb. Le fragment *Hind*III de 2,1 kpb a été purifié puis inséré dans le vecteur pZERO2 pour donner le plasmide pJMBYC02. La présence du gène de structure de la sakacine G dans pJMBYC02 a été démontrée par amplification PCR avec les amorces SakG01 et SakG02 puis par séquençage nucléotidique du fragment inséré dans pJMBYC02. Une stratégie voisine a été utilisée afin de déterminer la séquence complète du gène skgD. L'extrait plasmidique de *Lb. sakei* 2512 a été digéré par XbaI. Le produit de digestion a été inséré dans le plasmide pBluescript SK+. Les clones porteurs de la séquence d'intérêt ont été révélés au moyen d'une sonde radioactive préparée par PCR réalisée sur le plasmide pJMBYC02 à l'aide des oligonucléotides SakG03 (5' CCTTGGTCAGGCTATCG 3') (SEQ ID N°16) et SakG04 (5' ATCACCTTTTTGAATTACCC 3') (SEQ ID N°17).

L'analyse de la séquence nucléotidique complète de la région (3051 pb) a révélé l'existence de trois cadres ouverts de lecture complets *skgA1* et *skgA2* et *skgDc* et d'un tronqué, *skgI.* Les gènes supposés *skgA1 skgA2* et *skgI* sont orientés en sens inverse par rapport *à skgD.*

Chacun des cadres ouverts de lecture est précédé d'un site potentiel de fixation des ribosomes. Les gènes *skgA1* et *skgA2* codent tous les deux des protéines de 55 résidus d'acides aminés dont les séquences 19-55 sont totalement identiques. La séquence 19-52 correspond à la séquence de la sakacine G obtenue par microséquençage. La présence de 4 résidus cystéine en positions 9, 14 et 24 et C-terminale est à noter. De plus, la masse moléculaire calculée de ce peptide, de 3838,2 Da qui diffère de la masse moléculaire mesurée (3834,32 Da) de 4 Da montre la présence de deux ponts disulfures sur la sakacine G, comme cela a déjà été démontré pour d'autres bactériocines anti-*Listeria.*

Les séquences 1-18 des protéines SkgA1 et SkgA2 ne diffèrent que de 3 résidus et présentent de fortes homologies avec les peptides "leader" des bactériocines de classe II, qui sont impliquées dans le transport de ces peptides par des ABC-transporteurs spécifiques. En particulier le motif GG terminal est caractéristique de ces séquences leader et constitue le site de maturation de ces bactériocines. La comparaison des séquences nucléotidiques des gènes *skgA1* et *skgA2* montre également une identité de séquence de plus de 95% pour la partie des gènes codant la bactériocine mature.

Le cadre de lecture ouvert incomplet appelé *skgI* code une protéine de 52 résidus. La comparaison de cette séquence avec celles des banques de données montre de fortes homologies de SkgI avec les protéines dites d'immunité LccI et MesI. L'implication de MesI dans la protection vis à vis de la mésentéricine Y105 a été démontrée. On peut supposer que *skgI* code la protéine d'immunité à la sakacine G.

Le dernier gène *skgDc* code une protéine de 727 acides aminés. D'après les banques de données, SkgDc est très homologue de protéines de la famille des ABC-transporteurs et plus particulièrement des transporteurs de la pédiocine PA-1 : PedD ou PapD (Marugg et al., 1992; Appl Environ Microbiol 58, 2360-7; Motlagh et al., 1994, Lett Appl Microbiol 18, 305-12),de la sakacine P : SppT (Huhne et al., 1996, Microbiology 142, 1437-48), de la sakacine A : SapT (Axelsson and Holck, 1995, J Bacteriol 177, 2125-37) et de la mésentéricine Y105: MesD (Fremaux et al., 1995, Microbiology 141, 1637-45).

EXEMPLE 3 :

Spectre d'inhibition.

La sensibilité à la Sakacine G de 17 souches bactériennes a été testée par la méthode de test en puits (cf Matériels et Méthodes). Les résultats sont présentés dans le tableau 1 ci-après :

TABLEAU 1

| | Rayon des halos d'inhibition (mm) |
|---|---|
| *Lc. lactis* ATCC 11454 | 0 |
| *Ln. Paramesenteroides* DSM 20288 | 0 |
| *Ln. Mesenteroides* DSM 20484 | 0 |
| *Ln. Mesenteroides* DSM 20240 | 0 |
| *Lb. Delbrueckii* DSM 20081 | 0 |
| *Lb. Plantarum* DSM 20174 | 0 |
| *Lb brevis* DSM 20054 | 0 |
| *Lb. casei* DSM 20011 | 0 |
| *Lb. sakei* 2515 | 1 |
| *P. acidilactici* ENSAIA 583 | 0 |
| *P. cerevisiae* IP 5492 | 1 |
| *E. faecium* ENSAIA, 631 | 0 |
| *E. faecalis* IP 5430 | 2 |
| *E. faecalis* ENSAIA 636 | 1 |
| *E. durans* ENSAIA 630 | 2 |
| *L. inocua* 8811 | 3 |
| *L. ivanovi* BUG 496 | 6 |

Le spectre d'inhibition de cette bactériocine apparaît comme assez étroit et limité aux souches de *Lactobacillus sakei* et *Pediococcus cerevisiae* pour les bactéries lactiques. Ce peptide apparaît, comme les autres bactériocines de classe IIa, actif contre toutes les souches de *Listeria* testées, ainsi que contre les *Enterococcus faecalis* et *durans* mais pas contre *Enterococcus faecium.*

## Revendications

**1.** Polypeptide isolé, **caractérisé en ce qu'**il s'agit d'une bactériocine, dénommée Sakacine G de séquence ID N°12, issue de la souche *Lactobacillus sakei* 2512 déposée auprès de la Collection Nationale des Cultures de Microorganismes (CNCM) sous le numéro I-2479.

**2.** Polypeptide isolé selon la revendication 1, **caractérisé en ce qu'**il s'agit d'une bactériocine de classe IIa.

**3.** Pré-bactériocine isolée, **caractérisée en ce qu'**elle comprend la séquence ID N°2 et/ou la séquence ID N°4 ou pré-bactériocine homologue de séquence similaire à au moins 70% de la séquence SEQ ID N°2 ou N°4.

**4.** Bactériocine isolée, **caractérisée en ce qu'**elle comprend la séquence ID N°12 ou bactériocine homologue de séquence similaire à au moins 70% de la séquence SEQ ID N°12.

**5.** Acide nucléique de séquence nucléotidique codant pour un polypeptide selon l'une des revendications 1 à 4.

**6.** Acide nucléique selon la revendication 5, de séquence ID N°1 et/ou de séquence ID N°3 ou acide nucléique homologue de séquence similaire à au moins 70% de la séquence SEQ ID N°1 ou N°3.

**7.** Acide nucléique selon la revendication 5, de séquence d'acide nucléique SEQ ID N°15 ou acide nucléique homologue de séquence similaire à au moins 70% de la séquence SEQ ID N°15.

**8.** Acide nucléique selon la revendication 5, de séquence d'acide nucléique SEQ ID N°9 ou acide nucléique homologue de séquence similaire à au moins 70% de la séquence SEQ ID N°9.

**9.** Protéine d'immunité isolée, **caractérisée en ce qu'**elle comprend la séquence ID N°6 ou protéine d'immunité homologue de séquence similaire à au moins 70% de la séquence SEQ ID N°6.

**10.** ABC-transporteur isolé, **caractérisé en ce qu'**il comprend la séquence ID N°14 ou ABC-transporteur homologue de séquence similaire à au moins 70% de la séquence SEQ ID N°14.

**11.** Acide nucléique de séquence ID N°5 ou acide nucléique homologue de séquence similaire à au moins 70% de la séquence SEQ ID N°5, codant pour un polypeptide selon la revendication 9.

**12.** Acide nucléique de séquence ID N°13 ou acide nucléique homologue de séquence similaire à au moins 70% de la séquence SEQ ID N°13, codant pour un polypeptide selon la revendication 10.

**13.** Vecteur de clonage et/ou d'expression comprenant un acide nucléique selon l'une des revendications 5 à 8, 11, 12.

**14.** Cellule hôte transformée par un vecteur selon la revendication 13.

**15.** Cellule hôte selon la revendication 14, **caractérisée en ce qu'**il s'agit d'un microorganisme choisi parmi les *Lactococcus, Lactobacillus, Leuconostoc, Streptococcus, Pediococcus, Escherichia* ou d'une levure.

**16.** Procédé de production d'un polypeptide recombinant dans lequel un vecteur comprenant un acide nucléique selon l'une des revendications 5 à 8, 11, 12 est transféré dans une cellule hôte qui est mise en culture des conditions permettant l'expression d'un polypeptide selon l'une des revendications 1 à 4 et 9 à 10.

**17.** Utilisation d'un polypeptide selon l'une quelconque des revendications 1 à 4 comme agent actif contre des flores pathogènes ou indésirables dans la préparation de produits alimentaires.

**18.** Utilisation selon la revendication 17, **caractérisée en ce que** ledit polypeptide est mis en oeuvre pour inhiber la croissance et la propagation de *Listeria* dans les produits alimentaires.

**19.** Utilisation selon la revendication 18, **caractérisée en ce que** ledit polypeptide est mis en oeuvre pour inhiber la croissance et la propagation de *Listeria monocytogenes* dans les produits alimentaires.

**20.** Utilisation selon l'une quelconque des revendications 17 à 19, **caractérisée en ce que** ledit polypeptide est produit dans le produit alimentaire à partir de la souche *Lactobacillus Sakei* 2512 déposée auprès de la CNCM sous le numéro I-2479.

**21.** Utilisation de la souche *Lactobacillus Sakei* 2512 déposée auprès de la CNCM sous le numéro I-2479 dans des produits alimentaires pour y produire un polypeptide bactériocine selon l'une des revendications 1 à 4.

**22.** Composition bactériocine **caractérisée en ce qu'**elle comprend au moins un polypeptide selon l'une quelconque des revendications 1 à 4 ou la souche de *Lactobacillus Sakei* 2512 déposée auprès de la CNCM sous le numéro I-2479.

## Claims

**1.** Isolated polypeptide, **characterised in that** it is a bacteriocin, named Sakacin G of sequence ID No. 12, derived from the strain *Lactobacillus sakei* 2512 deposited with the Collection Nationale des Cultures de Microorganismes (CNCM) under deposit number I-2479.

**2.** Isolated polypeptide according to claim 1, **characterised in that** it is a class IIa bacteriocin.

**3.** Isolated pre-bacteriocin, **characterised in that** it comprises sequence ID No. 2 and/or sequence ID No. 4 or homologous pre-bacteriocin of sequence similar to at least 70% of sequence ID No. 2 or 4.

**4.** Isolated bacteriocin, **characterised in that** it comprises sequence ID No. 12 or homologous bacteriocin of sequence similar to at least 70% of sequence ID No. 12.

**5.** Nucleic acid of a nucleotide sequence encoding a polypeptide according to any one of claims 1 to 4.

**6.** Nucleic acid according to claim 5, of sequence ID No. 1 and/or sequence ID No. 3 or homologous nucleic acid of sequence similar to at least 70% of sequence ID No. 1 or 3.

**7.** Nucleic acid according to claim 5, of nucleic acid sequence SEQ ID No. 15 or homologous nucleic acid of sequence similar to at least 70% of sequence ID No. 15.

**8.** Nucleic acid according to claim 5, of nucleic acid sequence SEQ ID No. 9 or homologous nucleic acid of sequence similar to at least 70% of sequence ID No. 9.

**9.** Isolated immunity protein, **characterised in that** it comprises sequence ID No. 6 or homologous immunity protein of sequence similar to at least 70% of sequence ID No. 6.

**10.** Isolated ABC transporter, **characterised in that** it comprises sequence ID No. 14 or homologous ABC transporter of sequence similar to at least 70% of sequence ID No. 14.

**11.** Nucleic acid of sequence ID No. 5 or homologous nucleic acid of sequence similar to at least 70% of sequence ID No. 5, encoding a polypeptide according to claim 9.

**12.** Nucleic acid of sequence ID No. 13 or homologous nucleic acid of sequence similar to at least 70% of sequence ID No. 13, encoding a polypeptide according to claim 10.

**13.** Cloning and/or expression vector comprising a nucleic acid according to any one of claims 5 to 8, 11, 12.

**14.** Host cell transformed by a vector according to claim 13.

**15.** Host cell according to claim 14, **characterised in that** it is a microorganism chosen among *Lactococcus, Lactobacillus, Leuconostoc, Streptococcus, Pediococcus, Escherichia* or a yeast.

**16.** Process for the production of a recombinant polypeptide, in which a vector comprising a nucleic acid according to any one of claims 5 to 8, 11, 12 is transferred into a host cell which is cultured under conditions permitting the expression of a polypeptide according to any one of claims 1 to 4 and 9 to 10.

**17.** Use of a polypeptide according to any one of claims 1 to 4 as an active agent against pathogenic or undesirable flora in the preparation of food products.

**18.** Use according to claim 17, **characterised in that** said polypeptide is used to inhibit the growth and propagation of *Listeria* in food products.

**19.** Use according to claim 18, **characterised in that** said polypeptide is used to inhibit the growth and propagation of *Listeria monocytogenes* in food products.

**20.** Use according to any one of claims claim 17 to 19, **characterised in that** said polypeptide is produced in the food product from the strain *Lactobacillus sakei* 2512 deposited with the CNCM under deposit number I-2479.

**21.** Use of the strain *Lactobacillus sakei* 2512 deposited with the CNCM under deposit number 1-2479 in food products to produce therein a bacteriocin polypeptide according to any one of claims 1 to 4.

**22.** Bacteriocin composition, **characterised in that** it comprises at least one polypeptide according to any one of claims 1 to 4 or the strain *Lactobacillus sakei* 2512 deposited with the CNCM under deposit number I-2479.

**Patentansprüche**

**1.** Isoliertes Polypeptid, **dadurch gekennzeichnet, daß** es sich um ein Bacteriocin, Sakacine G genannt, mit der Sequenz ID NO: 12 handelt, das von dem Stamm Lactobacillus sakei 2512 stammt, der bei der Collection Nationale des Cultures de Microorganismes (CNCM) unter der Nr. I-2479 hinterlegt wurde.

**2.** Isoliertes Polypeptid nach Anspruch 1, **dadurch gekennzeichnet, daß** es sich um ein Bacteriocin der Klasse IIa handelt.

**3.** Isoliertes Pre-Bacteriocin, **dadurch gekennzeichnet, daß** es die Sequenz SEQ ID NO: 2 und/oder die Sequenz SEQ ID NO:4 oder homologes Pre-Bacteriocin mit einer Sequenz mit wenigstens 70 % Similarität zu der Sequenz SEQ ID NO:2 oder NO:4 umfaßt.

**4.** Isoliertes Bacteriocin, **dadurch gekennzeichnet, daß** es die SEQ ID NO:12 oder homologes Bacteriocin mit einer Sequenz mit wenigstens 70 Similarität zu der Sequenz SEQ ID NO:12 umfaßt.

**5.** Nukleinsäure mit der Nukleotidsequenz, die für ein Polypeptid nach den Ansprüchen 1 bis 4 codiert.

**6.** Nukleinsäure nach Anspruch 5 mit der Sequenz SEQ ID NO:1 und/oder mit der Sequenz SEQ ID NO:3 oder homologe Nukleinsäure mit einer Sequenz mit wenigstens 70 % Similarität zu der Sequenz SEQ ID NO:1 oder NO:3.

**7.** Nukleinsäure nach Anspruch 5 mit der Nukleinsäuresequenz SEQ ID NO:15 oder homologe Nukleinsäure mit einer Sequenz mit wenigstens 70 % Similarität zu der Sequenz SEQ ID NO:15.

**8.** Nukleinsäure nach Anspruch 5 mit der Nukleinsäuresequenz SEQ ID NO:9 oder homologe Nukleinsäure mit einer Sequenz mit wenigstens 70 % Similarität zu der Sequenz SEQ ID NO:9.

**9.** Isoliertes Immunitätsprotein, **dadurch gekennzeichnet, daß** es die Sequenz SEQ ID NO:6 oder homologes Immunitätsprotein mit einer Sequenz mit wenigstens 70 % Similarität zu der Sequenz SEQ ID NO:6 umfaßt.

**10.** Isolierter ABC-Transporter, **dadurch gekennzeichnet, daß** er die Sequenz SEQ ID NO:14 oder einen homologen ABC-Transporter mit einer Sequenz mit wenigstens 70 % Similarität zu der Sequenz SEQ ID NO:14 umfaßt.

**11.** Nukleinsäure mit der Sequenz SEQ ID NO:5 oder homologe Nukleinsäure mit einer Sequenz mit wenigstens 70 % Similarität zu der Sequenz SEQ ID NO:5, die für ein Polypeptid nach Anspruch 9 codiert.

**12.** Nukleinsäure mit der Sequenz SEQ ID NO:13 oder homologe Nukleinsäure mit einer Sequenz mit wenigstens 70 % Similarität zu der Sequenz SEQ ID NO:13, die für ein Polypeptid nach Anspruch 10 codier.

**13.** Klonierungs- und/oder Expressionsvektor, der eine Nukleinsäure nach einem der Ansprüche 5 bis 8, 11, 12 umfaßt.

**14.** Wirtszelle, die mit einem Vektor nach Anspruch 13 transformiert ist.

**15.** Wirtszelle nach Anspruch 14, **dadurch gekennzeichnet, daß** es sich um einen Mikroorganismus, ausgewählt unter Lactococcus, Lactobacillus, Leuconostoc, Streptococcus, Pediococcus, Escherichia und einer Hefe handelt.

**16.** Verfahren zur Herstellung eines rekombinanten Polypeptids, in dem ein Vektor, der eine Nukleinsäure nach einem der Ansprüche 5 bis 8, 11, 12 umfaßt, in eine Wirtszelle transferiert wird, die unter Bedingungen kultiviert wird, welche die Expression eines Polypeptids nach einem der Ansprüche 1 bis 4 und 9 bis 10 erlauben.

**17.** Verwendung eines Polypeptids nach einem der Ansprüche 1 bis 4 als Wirkstoff gegen pathogene oder unerwünschte Flora bei der Herstellung von Lebensmittelprodukten.

**18.** Verwendung nach Anspruch 17, **dadurch gekennzeichnet, daß** das Polypeptid verwendet wird, um das Wachstum und die Vermehrung von Listeria in Lebensmittelprodukten zu inhibieren.

**19.** Verwendung nach Anspruch 18, **dadurch gekennzeichnet, daß** das Peptid verwendet wird, um das Wachstum und die Vermehrung von Listeria monocytogenes in Lebensmittelprodukten zu inhibieren.

**20.** Verwendung nach einem der Ansprüche 17 bis 19, **dadurch gekennzeichnet, daß** das Polypeptid in dem Lebensmittelprodukt durch den Stamm Lactobacillus sakei 2512, der bei der CNCM unter der Nr. I-2479 hinterlegt wurde, produziert wird.

**21.** Verwendung des Stamms Lactobacillus sakei 2512, der bei der CNCM unter der Nr. I-2479 hinterlegt wurde, in Lebensmittelprodukten, um dort Bacteriocin-Polypeptid nach einem der Ansprüche 1 bis 4 zu produzieren.

**22.** Bacteriocin-Zusammensetzung, **dadurch gekennzeichnet, daß** sie wenigstens ein Polypeptid nach einem der Ansprüche 1 bis 4 oder den Stamm Lactobacillus sakei 2512, der bei der CNCM unter der Nr. I-2479 hinterlegt wurde, umfaßt.

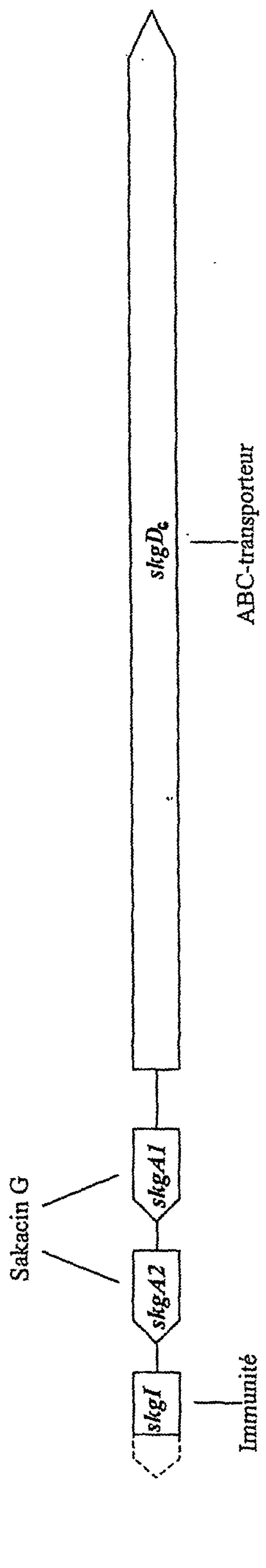
skgD_G
ABC-transporteur
Sakacin G
skgA1
skgA2
skgI
Immunité

FIG.1

3'
tcgaagccctaagaatcgatatagttaaaacgatatttgaaccctttcttgcagttattatagctcattaagtgacctaaaatgtcctgcgaaaagatcattcacgttctctcgaagctacaacaaaatctaagtcaaaattataacag
aaacaaacggtaaaattagtgcagaggaaaaaatatcattatttttttgtgttaatttaatcacgaaaaaatagaccattaattgtttgaggtactggcggtaatcgatctaccaaatgaggtgttcaggtacgaacgggggttaaatg
acttgtcggcactctcaatgtcgattgcggtaatggtatcataaatggtggacattatctaaagtaaagaacttaacaatccgaaaaacgcaaaaagtatttcttgtagaggtttaatataaaaaatcactaagaacttcaagacaac
attgcgtcttaaaaccttcttactcatgaacaatctttaaacggctaaatttattaattgtttggggtactggcggtaatcgatctactaaatggggtgttcaggtacgaacgggggttaaatgacttgttggtactctcaatgtcgattgtggt
aatggtatcataaatggtggacactacctaaaataaagaacctagcaattcgatgcacataagaagtaaaacttatggaggacaatttattaaaaatgtgctagtcacatcaagattacactttaacacagttcaaatcgtttatatat
aaaatccgtacctttttgaacgaaaattaagctgaactgatattgccatattatgaccataatgatataaacaaatcgaagtgttttttaatcctctgaatatataacaaattagacaactctatgttttttaatataacaagtgttcacctac
ttctactaacaccttagcgtcgaaacttatactaaaaatttttaaaaccaaggcttataagtgatagttttaacgctaagaatcgtttttggtcagttgttccctgatgataaaaacctgactatttccgacgtctccttgatttaaatcttcgctt
acgtaatgttcgactatacccgtagaaatttctacttttaaattacgatggttagtaacgtgtacaaaatttcgttccttttcaagacgtaatgatgcaacataaactacaaagctttctgaaaaattaataaccactgggtctgggttgttat
ccttaatgcctttagaggtttttcctaaaacttttacttacctgcccattaaagtattgtaaaagttttcctttcttgaaacaaagtctcttcgtctctttattgtcaaatgagttcaaataaggataaaactctgtcgtttttagggattataagacct
atcgaaagcggcgttatgataactactattaatcataacgtcctagtgaaaaaaatcttgttgaacatctatatatatatggtgtgtttttatacctatgtaacccctaatagagctaaacgaattaacctcggatagaaaatgtccggcat
tactgcataaaagtcttaaaaaatgattgatataaacctgttttagaaagatcttttaataaaatttaatataattagtggaaaaacttaatgggtacagaaagaagagttgtgcatctcaaccgctttatcagagagccaaatgtctac
gttcgttctaatatctacgaaaccgttcatgctaaaactgagagaaaaatctacaaacctacaaccaatgttagagttagcaagagcataaaaatttatgtttcaataaatactaaagagaccatagatatggccacatgagtcaat
attaaatacgcaaattttatgtaaattaccggacttggtatttagttaccttttacgtcgtaataacttaagacgttattagcttttgcattgaccgtatctttgacattttagtaattgaagtcttcttaaaaggatgttagtttagtgactatctaag
cttttaaaagaattgtcaaggaatgccatatgctatcgactggttcctgtcgttcgaaattttcacccaaacttcgattaagaatatcagaaatagaatacccgacctcgttaggttcaatactcccccttagagtgtcagccttctaataa
ccgaaaattacgaaatcattgtatgaaaaatttagggaatctcttataataattaaatgttggtttcgatgtttgacgttctcagcgattataatctgatttacttcataataatcacctaagactcaaattatccccccctgcgctgtcgagtt
gtttgaatttacccctatagcataaagttctacatcttaaatcaataccaatgcctagcttgcataacgtgttgtagttatatttttatgttttcttatcatcatgttgctaacaaccatactcgccaagaccctttaggtgtaatcgttttaattacc
aaccaaagatagttcggcctagtcctgtttataataatttaccatttaattagctattgtaactagcagtacgggactctgttagctaatgcatacatggtgtccttggccattacaagcgtccatgttaaaatcttttagaataatacgtctta
ttttctttaaatagataactatttcactttctccgtacatcctatcggctttaactatttctatatcttttaaaaggataccccatactatgtctataaaggcttgtaccctcaagttagagtcatccagtttttgtttctgaaagataacgttctagtga
cgactgtctcagattacaaaatgacaaactacttagttggtcatcaaacctgtgataatgactcgtcgcttattaactttggataacttaaatttactgttttgtaattataagcaacgtgtagctaacagtcaacgattcgtttgacttttata
atagcaatacctagtgccaccttaacaactttgtccaagcgtactatttaattataacctttacctataatatttcttaatacatgacacttctgcttctttttcttaaaatctattttgttttg 5'

FIG.2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **ENNAHAR S. et al.** *FEMS Microbiol. Rev.,* 2000, vol. 24, 85-106
- **JACK R. et al.** *Microbiol. Rev.,* 1995, vol. 59 (2), 171-200
- **DUFFES F. et al.** *J. Food Prot.,* 1999, vol. 62 (12), 1394-1403
- **TAGG J.R et al.** *Bacteriol. Rev.,* 1976, vol. 40, 722-756
- **HUGAS et al.** *Food Microbiol,* 1998, vol. 15, 639-650
- **MURIANA ; KLAENHAMMER.** *Appl. Environ. Microbiol.,* 1987, vol. 53, 553-560
- **HANAHAN.** *J. Mol. Biol.,* 1983, vol. 166, 557-80
- **MARUGG et al.** *Appl Environ Microbiol,* 1992, vol. 58, 2360-7
- **MOTLAGH et al.** *Lett Appl Microbiol,* 1994, vol. 18, 305-12
- **HUHNE et al.** *Microbiology,* 1996, vol. 142, 1437-48
- **HOLCK.** *J Bacteriol,* 1995, vol. 177, 2125-37
- **FREMAUX et al.** *Microbiology,* 1995, vol. 141, 1637-45

LISTE DE SEQUENCES

<110> RHODIA CHIMIE

<120> BACTERIOCINE ANTI-LISTERIA

<130>

<140>
<141>

<160> 17

<170> PatentIn Ver. 2.1

<210> 1
<211> 196
<212> ADN
<213> Lactobacillus sake

<220>
<221> CDS
<222> (20)..(187)

<400> 1

```
ttaacaggag gtattcaaa atg aag aat aca cgt agc tta acg atc caa gaa   52
                     Met Lys Asn Thr Arg Ser Leu Thr Ile Gln Glu
                      1               5                  10

ata aaa tcc atc aca ggt ggt aaa tac tat ggt aat ggt gtt agc tgt   100
Ile Lys Ser Ile Thr Gly Gly Lys Tyr Tyr Gly Asn Gly Val Ser Cys
            15                  20                  25

aac tct cat ggt tgt tca gta aat tgg ggg caa gca tgg act tgt ggg   148
Asn Ser His Gly Cys Ser Val Asn Trp Gly Gln Ala Trp Thr Cys Gly
        30                  35                  40

gta aat cat cta gct aat ggc ggt cat ggg gtt tgt taa ttatttaaa     196
Val Asn His Leu Ala Asn Gly Gly His Gly Val Cys
    45                  50                  55
```

<210> 2
<211> 55
<212> PRT
<213> Lactobacillus sake

<400> 2

```
Met Lys Asn Thr Arg Ser Leu Thr Ile Gln Glu Ile Lys Ser Ile Thr
 1               5                  10                  15

Gly Gly Lys Tyr Tyr Gly Asn Gly Val Ser Cys Asn Ser His Gly Cys
            20                  25                  30

Ser Val Asn Trp Gly Gln Ala Trp Thr Cys Gly Val Asn His Leu Ala
        35                  40                  45

Asn Gly Gly His Gly Val Cys
    50                  55
```

<210> 3
<211> 196
<212> ADN
<213> Lactobacillus sake

<220>
<221> CDS
<222> (20)..(187)

<400> 3

```
taatttggag atgttcttt atg aaa aac gca aaa agc cta aca att caa gaa  52
                     Met Lys Asn Ala Lys Ser Leu Thr Ile Gln Glu
                      1               5                   10

atg aaa tct att aca ggt ggt aaa tac tat ggt aat ggc gtt agc tgt   100
Met Lys Ser Ile Thr Gly Gly Lys Tyr Tyr Gly Asn Gly Val Ser Cys
            15                  20                  25

aac tct cac ggc tgt tca gta aat tgg ggg caa gca tgg act tgt gga   148
Asn Ser His Gly Cys Ser Val Asn Trp Gly Gln Ala Trp Thr Cys Gly
        30                  35                  40

gta aac cat cta gct aat ggc ggt cat gga gtt tgt taa ttaccagat     196
Val Asn His Leu Ala Asn Gly Gly His Gly Val Cys
    45                  50                  55
```

<210> 4
<211> 55
<212> PRT
<213> Lactobacillus sake

<400> 4

```
Met Lys Asn Ala Lys Ser Leu Thr Ile Gln Glu Met Lys Ser Ile Thr
 1               5                  10                  15

Gly Gly Lys Tyr Tyr Gly Asn Gly Val Ser Cys Asn Ser His Gly Cys
            20                  25                  30

Ser Val Asn Trp Gly Gln Ala Trp Thr Cys Gly Val Asn His Leu Ala
        35                  40                  45

Asn Gly Gly His Gly Val Cys
    50                  55
```

<210> 5
<211> 181
<212> ADN
<213> Lactobacillus sake

<220>
<221> CDS
<222> (24) .. (179)

<400> 5

```
ttaaaaaagg agacgtgatt aaa atg gca aac aaa gac aat att aaa act gaa 53
                          Met Ala Asn Lys Asp Asn Ile Lys Thr Glu
                           1               5                   10
```

```
tct aaa aac aac atc gaa gct ctc ttg cac tta cta gaa aag cgt cct    101
Ser Lys Asn Asn Ile Glu Ala Leu Leu His Leu Leu Glu Lys Arg Pro
                15                  20                  25

gta aaa tcc agt gaa tta ctc gat att att gac gtt ctt tcc caa gtt    149
Val Lys Ser Ser Glu Leu Leu Asp Ile Ile Asp Val Leu Ser Gln Val
            30                  35                  40

tat agc aaa att gat ata gct aag aat ccc ga                         181
Tyr Ser Lys Ile Asp Ile Ala Lys Asn Pro
        45                  50
```

<210> 6
<211> 52
<212> PRT
<213> Lactobacillus sake

<400> 6

```
Met Ala Asn Lys Asp Asn Ile Lys Thr Glu Ser Lys Asn Asn Ile Glu
 1               5                  10                  15

Ala Leu Leu His Leu Leu Glu Lys Arg Pro Val Lys Ser Ser Glu Leu
            20                  25                  30

Leu Asp Ile Ile Asp Val Leu Ser Gln Val Tyr Ser Lys Ile Asp Ile
        35                  40                  45

Ala Lys Asn Pro
    50
```

<210> 7
<211> 1203
<212> ADN
<213> Lactobacillus sake

<220>
<221> CDS
<222> (20) .. (1201)

<400> 7

```
aaattaggag acttatata ttg ttt aat ctg ttg aga tac aaa aaa tta tat  52
                     Leu Phe Asn Leu Leu Arg Tyr Lys Lys Leu Tyr
                      1               5                   10

tgt tca caa gtg gat gaa gat gat tgt gga atc gca gct ttg aat atg  100
Cys Ser Gln Val Asp Glu Asp Asp Cys Gly Ile Ala Ala Leu Asn Met
            15                  20                  25

att ttt aaa aat ttt ggt tcc gaa tat tca cta tca aaa ttg cga ttc  148
Ile Phe Lys Asn Phe Gly Ser Glu Tyr Ser Leu Ser Lys Leu Arg Phe
        30                  35                  40

tta gca aaa acc agt caa caa ggg act act att ttt gga ctg ata aag  196
Leu Ala Lys Thr Ser Gln Gln Gly Thr Thr Ile Phe Gly Leu Ile Lys
    45                  50                  55

gct gca gag gaa cta aat tta gaa gcg aat gca tta caa gct gat atg  244
```

```
Ala Ala Glu Glu Leu Asn Leu Glu Ala Asn Ala Leu Gln Ala Asp Met
 60                 65                  70                  75

ggc atc ttt aaa gat gaa aat tta atg cta cca atc att gca cat gtt    292
Gly Ile Phe Lys Asp Glu Asn Leu Met Leu Pro Ile Ile Ala His Val
                 80                  85                  90

tta aag caa gga aaa gtt ctg cat tac tac gtt gta ttt gat gtt tcg    340
Leu Lys Gln Gly Lys Val Leu His Tyr Tyr Val Val Phe Asp Val Ser
             95                 100                 105

aaa gac ttt tta att att ggt gac cca gac cca aca ata gga att acg    388
Lys Asp Phe Leu Ile Ile Gly Asp Pro Asp Pro Thr Ile Gly Ile Thr
        110                 115                 120

gaa atc tcc aaa aag gat ttt gaa aat gaa tgg acg ggt aat ttc ata    436
Glu Ile Ser Lys Lys Asp Phe Glu Asn Glu Trp Thr Gly Asn Phe Ile
    125                 130                 135

aca ttt tca aaa gga aag aac ttt gtt tca gag aag cag aga aat aac    484
Thr Phe Ser Lys Gly Lys Asn Phe Val Ser Glu Lys Gln Arg Asn Asn
140                 145                 150                 155

agt tta ctc aag ttt att cct att ttg aga cag caa aaa tcc cta ata    532
Ser Leu Leu Lys Phe Ile Pro Ile Leu Arg Gln Gln Lys Ser Leu Ile
                160                 165                 170

ttc tgg ata gct ttc gcc gca ata cta ttg atg ata att agt att gca    580
Phe Trp Ile Ala Phe Ala Ala Ile Leu Leu Met Ile Ile Ser Ile Ala
            175                 180                 185

gga tca ctt ttt tta gaa caa ctt gta gat ata tat ata cca cac aaa    628
Gly Ser Leu Phe Leu Glu Gln Leu Val Asp Ile Tyr Ile Pro His Lys
        190                 195                 200

aat atg gat aca ttg ggg att atc tcg att tgc tta att gga gcc tat    676
Asn Met Asp Thr Leu Gly Ile Ile Ser Ile Cys Leu Ile Gly Ala Tyr
    205                 210                 215

ctt tta cag gcc gta atg acg tat ttt cag aat ttt tta cta act ata    724
Leu Leu Gln Ala Val Met Thr Tyr Phe Gln Asn Phe Leu Leu Thr Ile
220                 225                 230                 235

ttt gga caa aat ctt tct aga aaa att att tta aat tat att aat cac    772
Phe Gly Gln Asn Leu Ser Arg Lys Ile Ile Leu Asn Tyr Ile Asn His
                240                 245                 250

ctt ttt gaa tta ccc atg tct ttc ttc tca aca cgt aga gtt ggc gaa    820
Leu Phe Glu Leu Pro Met Ser Phe Phe Ser Thr Arg Arg Val Gly Glu
            255                 260                 265

ata gtc tct cgg ttt aca gat gca agc aag att ata gat gct ttg gca    868
Ile Val Ser Arg Phe Thr Asp Ala Ser Lys Ile Ile Asp Ala Leu Ala
        270                 275                 280

agt acg att ttg act ctc ttt tta gat gtt tgg atg ttg gtt aca atc    916
Ser Thr Ile Leu Thr Leu Phe Leu Asp Val Trp Met Leu Val Thr Ile
    285                 290                 295

tca atc gtt ctc gta ttt tta aat aca aag tta ttt atg att tct ctg    964
Ser Ile Val Leu Val Phe Leu Asn Thr Lys Leu Phe Met Ile Ser Leu
```

```
300                 305                 310                 315

gta tct ata ccg gtg tac tca gtt ata att tat gcg ttt aaa aat aca    1012
Val Ser Ile Pro Val Tyr Ser Val Ile Ile Tyr Ala Phe Lys Asn Thr
                320                 325                 330

ttt aat ggc ctg aac cat aaa tca atg gaa aat gca gca tta ttg aat    1060
Phe Asn Gly Leu Asn His Lys Ser Met Glu Asn Ala Ala Leu Leu Asn
            335                 340                 345

tct gca ata atc gaa aac gta act ggc ata gaa act gta aaa tca tta    1108
Ser Ala Ile Ile Glu Asn Val Thr Gly Ile Glu Thr Val Lys Ser Leu
        350                 355                 360

act tca gaa gaa ttt tcc tac aat caa atc act gat aga ttc gaa aat    1156
Thr Ser Glu Glu Phe Ser Tyr Asn Gln Ile Thr Asp Arg Phe Glu Asn
    365                 370                 375

ttt ctt aac agt tcc tta cgg tat acg ata gct gac caa gga cag ca     1203
Phe Leu Asn Ser Ser Leu Arg Tyr Thr Ile Ala Asp Gln Gly Gln
380                 385                 390
```

<210> 8
<211> 394
<212> PRT
<213> Lactobacillus sake

<400> 8

```
Leu Phe Asn Leu Leu Arg Tyr Lys Lys Leu Tyr Cys Ser Gln Val Asp
 1               5                  10                  15

Glu Asp Asp Cys Gly Ile Ala Ala Leu Asn Met Ile Phe Lys Asn Phe
            20                  25                  30

Gly Ser Glu Tyr Ser Leu Ser Lys Leu Arg Phe Leu Ala Lys Thr Ser
        35                  40                  45

Gln Gln Gly Thr Thr Ile Phe Gly Leu Ile Lys Ala Ala Glu Glu Leu
    50                  55                  60

Asn Leu Glu Ala Asn Ala Leu Gln Ala Asp Met Gly Ile Phe Lys Asp
65                  70                  75                  80

Glu Asn Leu Met Leu Pro Ile Ile Ala His Val Leu Lys Gln Gly Lys
                85                  90                  95

Val Leu His Tyr Tyr Val Val Phe Asp Val Ser Lys Asp Phe Leu Ile
            100                 105                 110

Ile Gly Asp Pro Asp Pro Thr Ile Gly Ile Thr Glu Ile Ser Lys Lys
        115                 120                 125

Asp Phe Glu Asn Glu Trp Thr Gly Asn Phe Ile Thr Phe Ser Lys Gly
    130                 135                 140

Lys Asn Phe Val Ser Glu Lys Gln Arg Asn Asn Ser Leu Leu Lys Phe
145                 150                 155                 160

Ile Pro Ile Leu Arg Gln Gln Lys Ser Leu Ile Phe Trp Ile Ala Phe
                165                 170                 175
```

```
Ala Ala Ile Leu Leu Met Ile Ile Ser Ile Ala Gly Ser Leu Phe Leu
            180                 185                 190

Glu Gln Leu Val Asp Ile Tyr Ile Pro His Lys Asn Met Asp Thr Leu
        195                 200                 205

Gly Ile Ile Ser Ile Cys Leu Ile Gly Ala Tyr Leu Leu Gln Ala Val
    210                 215                 220

Met Thr Tyr Phe Gln Asn Phe Leu Leu Thr Ile Phe Gly Gln Asn Leu
225                 230                 235                 240

Ser Arg Lys Ile Ile Leu Asn Tyr Ile Asn His Leu Phe Glu Leu Pro
                245                 250                 255

Met Ser Phe Phe Ser Thr Arg Arg Val Gly Glu Ile Val Ser Arg Phe
            260                 265                 270

Thr Asp Ala Ser Lys Ile Ile Asp Ala Leu Ala Ser Thr Ile Leu Thr
        275                 280                 285

Leu Phe Leu Asp Val Trp Met Leu Val Thr Ile Ser Ile Val Leu Val
    290                 295                 300

Phe Leu Asn Thr Lys Leu Phe Met Ile Ser Leu Val Ser Ile Pro Val
305                 310                 315                 320

Tyr Ser Val Ile Ile Tyr Ala Phe Lys Asn Thr Phe Asn Gly Leu Asn
                325                 330                 335

His Lys Ser Met Glu Asn Ala Ala Leu Leu Asn Ser Ala Ile Ile Glu
            340                 345                 350

Asn Val Thr Gly Ile Glu Thr Val Lys Ser Leu Thr Ser Glu Glu Phe
        355                 360                 365

Ser Tyr Asn Gln Ile Thr Asp Arg Phe Glu Asn Phe Leu Asn Ser Ser
    370                 375                 380

Leu Arg Tyr Thr Ile Ala Asp Gln Gly Gln
385                 390
```

<210> 9
<211> 2042
<212> ADN
<213> Lactobacillus sake

<400> 9

```
agcttcggga ttcttagcta tatcaatttt gctataaact tgggaaagaa cgtcaataat 60
atcgagtaat tcactggatt ttacaggacg cttttctagt aagtgcaaga gagcttcgat 120
gttgttttta gattcagttt taatattgtc tttgtttgcc attttaatca cgtctccttt 180
tttatagtaa taaaaaaaac acaattaaat tagtgctttt ttatctggta attaacaaac 240
tccatgaccg ccattagcta gatggtttac tccacaagtc catgcttgcc cccaatttac 300
tgaacagccg tgagagttac agctaacgcc attaccatag tatttaccac ctgtaataga 360
tttcatttct tgaattgtta ggctttttgc gtttttcata aagaacatct ccaaattata 420
ttttttagtg attcttgaag ttctgttgta acgcagaatt ttggaagaat gagtacttgt 480
tagaaatttg ccgatttaaa taattaacaa accccatgac cgccattagc tagatgattt 540
accccacaag tccatgcttg cccccaattt actgaacaac catgagagtt acagctaaca 600
```

```
ccattaccat agtatttacc acctgtgatg gattttattt cttggatcgt taagctacgt 660
gtattcttca ttttgaatac ctcctgttaa ataattttta cacgatcagt gtagttctaa 720
tgtgaaattg tgtcaagttt agcaaatata tattttaggc atggaaaaac ttgcttttaa 780
ttcgacttga ctataacggt ataatactgg tattactata tttgtttagc ttcacaaaaa 840
aattaggaga cttatatatt gtttaatctg ttgagataca aaaaattata ttgttcacaa 900
gtggatgaag atgattgtgg aatcgcagct ttgaatatga tttttaaaaa ttttggttcc 960
gaatattcac tatcaaaatt gcgattctta gcaaaaacca gtcaacaagg gactactatt 1020
tttggactga taaaggctgc agaggaacta aatttagaag cgaatgcatt acaagctgat 1080
atgggcatct ttaaagatga aaatttaatg ctaccaatca ttgcacatgt tttaaagcaa 1140
ggaaaagttc tgcattacta cgttgtattt gatgtttcga aagacttttt aattattggt 1200
gacccagacc caacaatagg aattacggaa atctccaaaa aggattttga aaatgaatgg 1260
acgggtaatt tcataacatt ttcaaaagga aagaactttg tttcagagaa gcagagaaat 1320
aacagtttac tcaagtttat tcctattttg agacagcaaa aatccctaat attctggata 1380
gctttcgccg caatactatt gatgataatt agtattgcag gatcactttt tttagaacaa 1420
cttgtagata tatatatacc acacaaaaat atggatacat tggggattat ctcgatttgc 1500
ttaattggag cctatctttt acaggccgta atgacgtatt ttcagaattt tttactaact 1560
atatttggac aaaatctttc tagaaaaatt attttaaatt atattaatca ccttttttgaa 1620
ttacccatgt ctttcttctc aacacgtaga gttggcgaaa tagtctctcg gtttacagat 1680
gcaagcaaga ttatagatgc tttggcaagt acgattttga ctctcttttt agatgtttgg 1740
atgttggtta caatctcaat cgttctcgta tttttaaata caaagttatt tatgatttct 1800
ctggtatcta taccggtgta ctcagttata atttatgcgt ttaaaaatac atttaatggc 1860
ctgaaccata aatcaatgga aaatgcagca ttattgaatt ctgcaataat cgaaaacgta 1920
actggcatag aaactgtaaa atcattaact tcagaagaat tttcctacaa tcaaatcact 1980
gatagattcg aaaattttct taacagttcc ttacggtata cgatagctga ccaaggacag 2040
ca                                                                2042
```

<210> 10
<211> 20
<212> ADN
<213> Lactobacillus sake

<400> 10
aartattatg gnaayggngt 20

<210> 11
<211> 20
<212> ADN
<213> Lactobacillus sake

<400> 11
acatgatgnc cnccrttngc 20

<210> 12
<211> 37

<212> PRT
<213> Lactobacillus sake

<400> 12

```
Lys Tyr Tyr Gly Asn Gly Val Ser Cys Asn Ser His Gly Cys Ser Val
1               5                   10                  15

Asn Trp Gly Gln Ala Trp Thr Cys Gly Val Asn His Leu Ala Asn Gly
            20                  25                  30

Gly His Gly Val Cys
        35
```

<210> 13
<211> 2214
<212> ADN
<213> lactobacillus sake

<220>
<221> CDS
<222> (20) .. (2200)

<400> 13

```
aaattaggag acttatata ttg ttt aat ctg ttg aga tac aaa aaa tta tat  52
                     Leu Phe Asn Leu Leu Arg Tyr Lys Lys Leu Tyr
                      1               5                  10

tgt tca caa gtg gat gaa gat gat tgt gga atc gca gct ttg aat atg  100
Cys Ser Gln Val Asp Glu Asp Asp Cys Gly Ile Ala Ala Leu Asn Met
            15                  20                  25

att ttt aaa aat ttt ggt tcc gaa tat tca cta tca aaa ttg cga ttc  148
Ile Phe Lys Asn Phe Gly Ser Glu Tyr Ser Leu Ser Lys Leu Arg Phe
        30                  35                  40

tta gca aaa acc agt caa caa ggg act act att ttt gga ctg ata aag  196
Leu Ala Lys Thr Ser Gln Gln Gly Thr Thr Ile Phe Gly Leu Ile Lys
    45                  50                  55

gct gca gag gaa cta aat tta gaa gcg aat gca tta caa gct gat atg  244
Ala Ala Glu Glu Leu Asn Leu Glu Ala Asn Ala Leu Gln Ala Asp Met
 60                  65                  70                  75

ggc atc ttt aaa gat gaa aat tta atg cta cca atc att gca cat gtt  292
Gly Ile Phe Lys Asp Glu Asn Leu Met Leu Pro Ile Ile Ala His Val
                80                  85                  90

tta aag caa gga aaa gtt ctg cat tac tac gtt gta ttt gat gtt tcg  340
Leu Lys Gln Gly Lys Val Leu His Tyr Tyr Val Val Phe Asp Val Ser
            95                  100                 105

aaa gac ttt tta att att ggt gac cca gac cca aca ata gga att acg  388
Lys Asp Phe Leu Ile Ile Gly Asp Pro Asp Pro Thr Ile Gly Ile Thr
        110                 115                 120

gaa atc tcc aaa aag gat ttt gaa aat gaa tgg acg ggt aat ttc ata  436
Glu Ile Ser Lys Lys Asp Phe Glu Asn Glu Trp Thr Gly Asn Phe Ile
    125                 130                 135

aca ttt tca aaa gga aag aac ttt gtt tca gag aag cag aga aat aac  484
Thr Phe Ser Lys Gly Lys Asn Phe Val Ser Glu Lys Gln Arg Asn Asn
140                 145                 150                 155

agt tta ctc aag ttt att cct att ttg aga cag caa aaa tcc cta ata  532
Ser Leu Leu Lys Phe Ile Pro Ile Leu Arg Gln Gln Lys Ser Leu Ile
                160                 165                 170

ttc tgg ata gct ttc gcc gca ata cta ttg atg ata att agt att gca  580
Phe Trp Ile Ala Phe Ala Ala Ile Leu Leu Met Ile Ile Ser Ile Ala
            175                 180                 185

gga tca ctt ttt tta gaa caa ctt gta gat ata tat ata cca cac aaa  628
Gly Ser Leu Phe Leu Glu Gln Leu Val Asp Ile Tyr Ile Pro His Lys
        190                 195                 200
```

```
aat atg gat aca ttg ggg att atc tcg att tgc tta att gga gcc tat    676
Asn Met Asp Thr Leu Gly Ile Ile Ser Ile Cys Leu Ile Gly Ala Tyr
    205                 210                 215

ctt tta cag gcc gta atg acg tat ttt cag aat ttt tta cta act ata    724
Leu Leu Gln Ala Val Met Thr Tyr Phe Gln Asn Phe Leu Leu Thr Ile
220                 225                 230                 235

ttt gga caa aat ctt tct aga aaa att att tta aat tat att aat cac    772
Phe Gly Gln Asn Leu Ser Arg Lys Ile Ile Leu Asn Tyr Ile Asn His
                240                 245                 250

ctt ttt gaa tta ccc atg tct ttc ttc tca aca cgt aga gtt ggc gaa    820
Leu Phe Glu Leu Pro Met Ser Phe Phe Ser Thr Arg Arg Val Gly Glu
            255                 260                 265

ata gtc tct cgg ttt aca gat gca agc aag att ata gat gct ttg gca    868
Ile Val Ser Arg Phe Thr Asp Ala Ser Lys Ile Ile Asp Ala Leu Ala
        270                 275                 280

agt acg att ttg act ctc ttt tta gat gtt tgg atg ttg gtt aca atc    916
Ser Thr Ile Leu Thr Leu Phe Leu Asp Val Trp Met Leu Val Thr Ile
    285                 290                 295

tca atc gtt ctc gta ttt tta aat aca aag tta ttt atg att tct ctg    964
Ser Ile Val Leu Val Phe Leu Asn Thr Lys Leu Phe Met Ile Ser Leu
300                 305                 310                 315

gta tct ata ccg gtg tac tca gtt ata att tat gcg ttt aaa aat aca    1012
Val Ser Ile Pro Val Tyr Ser Val Ile Ile Tyr Ala Phe Lys Asn Thr
                320                 325                 330

ttt aat ggc ctg aac cat aaa tca atg gaa aat gca gca tta ttg aat    1060
Phe Asn Gly Leu Asn His Lys Ser Met Glu Asn Ala Ala Leu Leu Asn
            335                 340                 345

tct gca ata atc gaa aac gta act ggc ata gaa act gta aaa tca tta    1108
Ser Ala Ile Ile Glu Asn Val Thr Gly Ile Glu Thr Val Lys Ser Leu
        350                 355                 360

act tca gaa gaa ttt tcc tac aat caa atc act gat aga ttc gaa aat    1156
Thr Ser Glu Glu Phe Ser Tyr Asn Gln Ile Thr Asp Arg Phe Glu Asn
    365                 370                 375

ttt ctt aac agt tcc tta cgg tat acg ata gct gac caa gga cag caa    1204
Phe Leu Asn Ser Ser Leu Arg Tyr Thr Ile Ala Asp Gln Gly Gln Gln
380                 385                 390                 395

gct tta aaa gtg ggt ttg aag cta att ctt ata gtc ttt atc tta tgg    1252
Ala Leu Lys Val Gly Leu Lys Leu Ile Leu Ile Val Phe Ile Leu Trp
                400                 405                 410

gct gga gca atc caa gtt atg agg ggg aat ctc aca gtc gga aga tta    1300
Ala Gly Ala Ile Gln Val Met Arg Gly Asn Leu Thr Val Gly Arg Leu
            415                 420                 425

ttg gct ttt aat gct tta gta aca tac ttt tta aat ccc tta gag aat    1348
Leu Ala Phe Asn Ala Leu Val Thr Tyr Phe Leu Asn Pro Leu Glu Asn
        430                 435                 440
```

```
att att aat tta caa cca aag cta caa act gca aga gtc gct aat att   1396
Ile Ile Asn Leu Gln Pro Lys Leu Gln Thr Ala Arg Val Ala Asn Ile
    445                 450                 455

aga cta aat gaa gta tta tta gtg gat tct gag ttt aat agg ggg gga   1444
Arg Leu Asn Glu Val Leu Leu Val Asp Ser Glu Phe Asn Arg Gly Gly
460                 465                 470                 475

cgc gac agc tca aca aac tta aat ggg gat atc gta ttt caa gat gta   1492
Arg Asp Ser Ser Thr Asn Leu Asn Gly Asp Ile Val Phe Gln Asp Val
                480                 485                 490

gaa ttt agt tat ggt tac gga tcg aac gta ttg cac aac atc aat ata   1540
Glu Phe Ser Tyr Gly Tyr Gly Ser Asn Val Leu His Asn Ile Asn Ile
            495                 500                 505

aaa ata caa aag aat agt agt aca acg att gtt ggt atg agc ggt tct   1588
Lys Ile Gln Lys Asn Ser Ser Thr Thr Ile Val Gly Met Ser Gly Ser
        510                 515                 520

ggg aaa tcc aca tta gca aaa tta atg gtt ggt ttc tat caa gcc gga   1636
Gly Lys Ser Thr Leu Ala Lys Leu Met Val Gly Phe Tyr Gln Ala Gly
    525                 530                 535

tca gga caa ata tta tta aat ggt aaa tta atc gat aac att gat cgt   1684
Ser Gly Gln Ile Leu Leu Asn Gly Lys Leu Ile Asp Asn Ile Asp Arg
540                 545                 550                 555

cat gcc ctg aga caa tcg att acg tat gta cca cag gaa ccg gta atg   1732
His Ala Leu Arg Gln Ser Ile Thr Tyr Val Pro Gln Glu Pro Val Met
                560                 565                 570

ttc gca ggt aca att tta gaa aat ctt att atg cag aat aaa aga aat   1780
Phe Ala Gly Thr Ile Leu Glu Asn Leu Ile Met Gln Asn Lys Arg Asn
            575                 580                 585

tta tct att gat aaa gtg aaa gag gca tgt agg ata gcc gaa att gat   1828
Leu Ser Ile Asp Lys Val Lys Glu Ala Cys Arg Ile Ala Glu Ile Asp
        590                 595                 600

aaa gat ata gaa aat ttt cct atg ggg tat gat aca gat att tcc gaa   1876
Lys Asp Ile Glu Asn Phe Pro Met Gly Tyr Asp Thr Asp Ile Ser Glu
    605                 610                 615

cat ggg agt tca atc tca gta ggt caa aaa caa aga ctt tct att gca   1924
His Gly Ser Ser Ile Ser Val Gly Gln Lys Gln Arg Leu Ser Ile Ala
620                 625                 630                 635

aga tca ctg ctg aca gag tct aat gtt tta ctg ttt gat gaa tca acc   1972
Arg Ser Leu Leu Thr Glu Ser Asn Val Leu Leu Phe Asp Glu Ser Thr
                640                 645                 650

agt agt ttg gac act att act gag cag cga ata att gaa aac cta ttg   2020
Ser Ser Leu Asp Thr Ile Thr Glu Gln Arg Ile Ile Glu Asn Leu Leu
            655                 660                 665

aat tta aat gac aaa aca tta ata ttc gtt gca cat cga ttg tca gtt   2068
Asn Leu Asn Asp Lys Thr Leu Ile Phe Val Ala His Arg Leu Ser Val
        670                 675                 680
```

```
gct aag caa act gaa aat att atc gtt atg gat cac ggt gga att gtt    2116
Ala Lys Gln Thr Glu Asn Ile Ile Val Met Asp His Gly Gly Ile Val
    685                 690                 695

gaa aca ggt tcg cat gat aaa tta ata ttg gaa aat gga tat tat aaa    2164
Glu Thr Gly Ser His Asp Lys Leu Ile Leu Glu Asn Gly Tyr Tyr Lys
700                 705                 710                 715

gaa tta tgt act gtg aag acg aag aaa aaa gaa ttt tagataaaac aaaa    2214
Glu Leu Cys Thr Val Lys Thr Lys Lys Lys Glu Phe
                720                 725
```

<210> 14
<211> 727
<212> PRT
<213> lactobacillus sake

<400> 14

```
Leu Phe Asn Leu Leu Arg Tyr Lys Lys Leu Tyr Cys Ser Gln Val Asp
 1               5                   10                  15

Glu Asp Asp Cys Gly Ile Ala Ala Leu Asn Met Ile Phe Lys Asn Phe
            20                  25                  30

Gly Ser Glu Tyr Ser Leu Ser Lys Leu Arg Phe Leu Ala Lys Thr Ser
        35                  40                  45

Gln Gln Gly Thr Thr Ile Phe Gly Leu Ile Lys Ala Ala Glu Glu Leu
    50                  55                  60

Asn Leu Glu Ala Asn Ala Leu Gln Ala Asp Met Gly Ile Phe Lys Asp
65                  70                  75                  80

Glu Asn Leu Met Leu Pro Ile Ile Ala His Val Leu Lys Gln Gly Lys
                85                  90                  95

Val Leu His Tyr Tyr Val Val Phe Asp Val Ser Lys Asp Phe Leu Ile
            100                 105                 110

Ile Gly Asp Pro Asp Pro Thr Ile Gly Ile Thr Glu Ile Ser Lys Lys
        115                 120                 125

Asp Phe Glu Asn Glu Trp Thr Gly Asn Phe Ile Thr Phe Ser Lys Gly
    130                 135                 140

Lys Asn Phe Val Ser Glu Lys Gln Arg Asn Asn Ser Leu Leu Lys Phe
145                 150                 155                 160

Ile Pro Ile Leu Arg Gln Gln Lys Ser Leu Ile Phe Trp Ile Ala Phe
                165                 170                 175

Ala Ala Ile Leu Leu Met Ile Ile Ser Ile Ala Gly Ser Leu Phe Leu
            180                 185                 190

Glu Gln Leu Val Asp Ile Tyr Ile Pro His Lys Asn Met Asp Thr Leu
        195                 200                 205

Gly Ile Ile Ser Ile Cys Leu Ile Gly Ala Tyr Leu Leu Gln Ala Val
    210                 215                 220
```

Met Thr Tyr Phe Gln Asn Phe Leu Leu Thr Ile Phe Gly Gln Asn Leu
225 230 235 240

Ser Arg Lys Ile Ile Leu Asn Tyr Ile Asn His Leu Phe Glu Leu Pro
245 250 255

Met Ser Phe Phe Ser Thr Arg Arg Val Gly Glu Ile Val Ser Arg Phe
260 265 270

Thr Asp Ala Ser Lys Ile Ile Asp Ala Leu Ala Ser Thr Ile Leu Thr
275 280 285

Leu Phe Leu Asp Val Trp Met Leu Val Thr Ile Ser Ile Val Leu Val
290 295 300

Phe Leu Asn Thr Lys Leu Phe Met Ile Ser Leu Val Ser Ile Pro Val
305 310 315 320

Tyr Ser Val Ile Ile Tyr Ala Phe Lys Asn Thr Phe Asn Gly Leu Asn
325 330 335

His Lys Ser Met Glu Asn Ala Ala Leu Leu Asn Ser Ala Ile Ile Glu
340 345 350

Asn Val Thr Gly Ile Glu Thr Val Lys Ser Leu Thr Ser Glu Glu Phe
355 360 365

Ser Tyr Asn Gln Ile Thr Asp Arg Phe Glu Asn Phe Leu Asn Ser Ser
370 375 380

Leu Arg Tyr Thr Ile Ala Asp Gln Gly Gln Gln Ala Leu Lys Val Gly
385 390 395 400

Leu Lys Leu Ile Leu Ile Val Phe Ile Leu Trp Ala Gly Ala Ile Gln
405 410 415

Val Met Arg Gly Asn Leu Thr Val Gly Arg Leu Leu Ala Phe Asn Ala
420 425 430

Leu Val Thr Tyr Phe Leu Asn Pro Leu Glu Asn Ile Ile Asn Leu Gln
435 440 445

Pro Lys Leu Gln Thr Ala Arg Val Ala Asn Ile Arg Leu Asn Glu Val
450 455 460

Leu Leu Val Asp Ser Glu Phe Asn Arg Gly Gly Arg Asp Ser Ser Thr
465 470 475 480

Asn Leu Asn Gly Asp Ile Val Phe Gln Asp Val Glu Phe Ser Tyr Gly
485 490 495

Tyr Gly Ser Asn Val Leu His Asn Ile Asn Ile Lys Ile Gln Lys Asn
500 505 510

Ser Ser Thr Thr Ile Val Gly Met Ser Gly Ser Gly Lys Ser Thr Leu
515 520 525

Ala Lys Leu Met Val Gly Phe Tyr Gln Ala Gly Ser Gly Gln Ile Leu
530 535 540

```
Leu Asn Gly Lys Leu Ile Asp Asn Ile Asp Arg His Ala Leu Arg Gln
545                 550                 555                 560

Ser Ile Thr Tyr Val Pro Gln Glu Pro Val Met Phe Ala Gly Thr Ile
                565                 570                 575

Leu Glu Asn Leu Ile Met Gln Asn Lys Arg Asn Leu Ser Ile Asp Lys
            580                 585                 590

Val Lys Glu Ala Cys Arg Ile Ala Glu Ile Asp Lys Asp Ile Glu Asn
        595                 600                 605

Phe Pro Met Gly Tyr Asp Thr Asp Ile Ser Glu His Gly Ser Ser Ile
    610                 615                 620

Ser Val Gly Gln Lys Gln Arg Leu Ser Ile Ala Arg Ser Leu Leu Thr
625                 630                 635                 640

Glu Ser Asn Val Leu Leu Phe Asp Glu Ser Thr Ser Ser Leu Asp Thr
                645                 650                 655

Ile Thr Glu Gln Arg Ile Ile Glu Asn Leu Leu Asn Leu Asn Asp Lys
            660                 665                 670

Thr Leu Ile Phe Val Ala His Arg Leu Ser Val Ala Lys Gln Thr Glu
        675                 680                 685

Asn Ile Ile Val Met Asp His Gly Gly Ile Val Glu Thr Gly Ser His
    690                 695                 700

Asp Lys Leu Ile Leu Glu Asn Gly Tyr Tyr Lys Glu Leu Cys Thr Val
705                 710                 715                 720

Lys Thr Lys Lys Lys Glu Phe
                725
```

<210> 15
<211> 3055
<212 ADN
<213> lactobacillus sake

<400> 15

agcttcggga ttcttagcta tatcaatttt gctataaact tgggaaagaa cgtcaataat 60
atcgagtaat tcactggatt ttacaggacg cttttctagt aagtgcaaga gagcttcgat 120
gttgttttta gattcagttt taatattgtc tttgtttgcc attttaatca cgtctccttt 180
tttatagtaa taaaaaaaac acaattaaat tagtgctttt ttatctggta attaacaaac 240
tccatgaccg ccattagcta gatggtttac tccacaagtc catgcttgcc cccaatttac 300
tgaacagccg tgagagttac agctaacgcc attaccatag tatttaccac ctgtaataga 360
tttcatttct tgaattgtta ggctttttgc gtttttcata aagaacatct ccaaattata 420
tttttagtg attcttgaag ttctgttgta acgcagaatt ttggaagaat gagtacttgt 480
tagaaatttg ccgatttaaa taattaacaa accccatgac cgccattagc tagatgattt 540
accccacaag tccatgcttg cccccaattt actgaacaac catgagagtt acagctaaca 600
ccattaccat agtatttacc acctgtgatg gattttattt cttggatcgt taagctacgt 660
gtattcttca ttttgaatac ctcctgttaa ataattttta cacgatcagt gtagttctaa 720
tgtgaaattg tgtcaagttt agcaaatata tattttaggc atggaaaaac ttgcttttaa 780
ttcgacttga ctataacggt ataatactgg tattactata tttgtttagc ttcacaaaaa 840
aattaggaga cttatatatt gtttaatctg ttgagataca aaaaattata ttgttcacaa 900
gtggatgaag atgattgtgg aatcgcagct ttgaatatga tttttaaaaa ttttggttcc 960
gaatattcac tatcaaaatt gcgattctta gcaaaaacca gtcaacaagg gactactatt 1020

tttggactga taaaggctgc agaggaacta aatttagaag cgaatgcatt acaagctgat 1080
atgggcatct ttaaagatga aaatttaatg ctaccaatca ttgcacatgt tttaaagcaa 1140
ggaaaagttc tgcattacta cgttgtattt gatgtttcga aagacttttt aattattggt 1200
gacccagacc caacaatagg aattacggaa atctccaaaa aggattttga aaatgaatgg 1260
acgggtaatt tcataacatt ttcaaaagga aagaactttg tttcagagaa gcagagaaat 1320
aacagtttac tcaagtttat tcctattttg agacagcaaa aatccctaat attctggata 1380
gctttcgccg caatactatt gatgataatt agtattgcag gatcactttt tttagaacaa 1440
cttgtagata tatatatacc acacaaaaat atggatacat tggggattat ctcgatttgc 1500
ttaattggag cctatctttt acaggccgta atgacgtatt ttcagaattt tttactaact 1560
atatttggac aaaatctttc tagaaaaatt attttaaatt atattaatca ccttttttgaa 1620
ttacccatgt ctttcttctc aacacgtaga gttggcgaaa tagtctctcg gtttacagat 1680
gcaagcaaga ttatagatgc tttggcaagt acgatttgga ctctcttttt agatgtttgg 1740
atgttggtta caatctcaat cgttctcgta tttttaaata caaagttatt tatgatttct 1800
ctggtatcta taccggtgta ctcagttata atttatgcgt ttaaaaatac atttaatggc 1860
ctgaaccata aatcaatgga aaatgcagca ttattgaatt ctgcaataat cgaaaacgta 1920
actggcatag aaactgtaaa atcattaact tcagaagaat ttcctacaa tcaaatcact 1980
gatagattcg aaaattttct taacagttcc ttacggtata cgatagctga ccaaggacag 2040
caagctttaa aagtgggttt gaagctaatt cttatagtct ttatcttatg ggctggagca 2100
atccaagtta tgaggggggaa tctcacagtc ggaagattat tggcttttaa tgctttagta 2160
acatactttt taaatccctt agagaatatt attaatttac aaccaaagct acaaactgca 2220
agagtcgcta atattagact aaatgaagta ttattagtgg attctgagtt taatagggggg 2280
ggacgcgaca gctcaacaaa cttaaatggg gatatcgtat ttcaagatgt agaatttagt 2340
tatggttacg gatcgaacgt attgcacaac atcaatataa aaatacaaaa gaatagtagt 2400
acaacgattg ttggtatgag cggttctggg aaatccacat tagcaaaatt aatggttggt 2460
ttctatcaag ccggatcagg acaaatatta ttaaatggta aattaatcga taacattgat 2520
cgtcatgccc tgagacaatc gattacgtat gtaccacagg aaccggtaat gttcgcaggt 2580
acaattttag aaaatcttat tatgcagaat aaaagaaatt tatctattga taaagtgaaa 2640
gaggcatgta ggatagccga aattgataaa gatatagaaa attttcctat ggggtatgat 2700
acagatattt ccgaacatgg gagttcaatc tcagtaggtc aaaaacaaag actttctatt 2760
gcaagatcac tgctgacaga gtctaatgtt ttactgtttg atgaatcaac cagtagtttg 2820
gacactatta ctgagcagcg aataattgaa aacctattga atttaaatga caaaacatta 2880
atattcgttg cacatcgatt gtcagttgct aagcaaactg aaaatattat cgttatggat 2940
cacggtggaa ttgttgaaac aggttcgcat gataaattaa tattggaaaa tggatattat 3000
aaagaattat gtactgtgaa gacgaagaaa aaagaatttt agataaaaca aaaac 3055

<210> 16
<211> 17

<212> ADN
<213> lactobacillus sake

<400> 16
ccttggtcag gctatcg 17

<210> 17
<211> 20
<212> ADN
<213> lactobacillus sake

<400> 17
atcacctttt tgaattaccc 20